# EUROPEAN PATENT SPECIFICATION

(11) **EP 3 992 977 B1**
(45) Date of publication and mention of the grant of the patent: **11.11.2026**
(21) Application number: 20850434.0
(22) Date of filing: 18.03.2020
(51) Int. Cl.: G16H 10/60, G16H 10/20, G16H 40/40, G16H 40/63, A61B 5/145, A61B 5/00, H04M 1/725, A61B 5/1495, H04M 1/72412

(54) **METHOD FOR MANAGING CALIBRATION INFORMATION IN CONTINUOUS BLOOD GLUCOSE MEASUREMENT SYSTEM**
VERFAHREN ZUR VERWALTUNG VON KALIBRATIONSINFORMATIONEN IN EINEM KONTINUIERLICHEN BLUTZUCKERMESSSYSTEM
PROCÉDÉ DE GESTION D'INFORMATIONS DE CALIBRATION DANS UN SYSTÈME DE MESURE EN CONTINU DE LA GLYCÉMIE

(30) Priority: 08.08.2019 KR 20190096796
(43) Date of publication of application: 04.05.2022
(73) Proprietor: i-Sens, Inc., Seoul 06646 (KR)
(72) Inventor: LEE, Seung Chun, Seoul 06646 (KR)
(74) Representative: BCKIP Part mbB
(86) International application number: PCT/KR2020/003711
(87) International publication number: WO 2021/025261

(56) References cited:
- EP-A2- 2 327 984
- JP-A- 2011 139 942
- JP-A- 2014 158 970
- JP-A- 2018 509 947
- JP-B2- S5 837 570
- KR-B1- 101 998 517
- US-A1- 2010 191 086
- US-A1- 2016 210 099
- US-A1- 2017 286 194

## Description

### TECHNICAL FIELD

The present invention is generally related to a method for managing calibration information in a continuous blood glucose monitoring system, in more detail, a method for managing calibration information providing, to a sensor transmitter, calibration information periodically acquired and time points of acquiring the calibration information and receiving information of the calibration information and the time point of acquiring the calibration information at the time of performing communication connection between the a sensor transmitter and a communication terminal, thereby not needing to repeat a stabilization process or initial calibration of the sensor transmitter, and determining whether additional calibration information is needed or not at the time of performing communication connection and acquiring the additional calibration information or outputting biometric information using the received calibration information.

### BACKGROUND

Diabetes is a chronic medical condition that is common in modern people, and in the Republic of Korea, there are 2 million diabetes patients, about 5% of the total population.

Diabetes occurs when the absolute level of the sugar level in blood is high due to the absolute deficiency or relative insufficiency of insulin, produced by the pancreas, caused by various reasons such as obesity, stress, poor eating habits, and inherited hereditary factors and imbalance regarding glucose in the blood.

The blood usually contains a certain concentration of glucose, and tissue cells gain energy from the glucose.

However, when the glucose is increased excessively more than needed, the glucose cannot be properly stored in the liver, muscle, or adipose tissue and is accumulated in the blood, because of this, patients with diabetes maintain a much higher blood glucose level than normal people, and as excessive blood glucose passes through the tissues and is discharged into the urine, it results in deficiency of glucose, which is absolutely necessary for all tissues of the body, thereby causing abnormalities in respective body tissues.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown, and further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

In order to manage diabetes, the continuous measurement of blood glucose is required, and therefore the demand for devices related to blood glucose measurement is steadily increasing. It has been confirmed through various studies that the occurrence of complications of diabetes is significantly reduced when diabetic patients strictly control blood glucose. Accordingly, it is very important for diabetics to regularly measure blood glucose in order to manage blood glucose.

In general, a finger prick method is mainly used for blood glucose management by diabetic patients and this type of blood sugar collection system helps the diabetic patients in managing their blood glucose, but it is difficult to accurately identify the blood glucose levels which are being frequently changed because it shows only the result at the time of the measurement. In addition, the finger prick type blood glucose device needs to collect blood several times from time to time even in a day, so diabetics have a burden on blood collection.

Diabetics patients generally experience hyperglycemia and hypoglycemia, and an emergency may occur in the hypoglycemic conditions. The patients may become unconscious or die if a hypoglycemic condition lasts for an extended period of time without the supply of sugar. Accordingly, rapid discovery of the hypoglycemic condition is critically important for diabetics. However, blood-collecting type glucose monitoring devices intermittently measuring glucose have limited ability to accurately measure blood glucose levels.

To overcome such a drawback, continuous glucose monitoring systems (CGMSs) inserted into the human body to measure a blood glucose level every few minutes have been developed, and therefore easily perform the management of diabetics and responses to an emergency situation.

A continuous blood glucose monitoring system comprises a sensor transmitter attached to human body of a user, extracting body fluid, and measuring blood glucose, and a communication terminal displaying a received blood glucose level. The sensor transmitter generates blood glucose information by measuring the blood glucose of the user for a certain period, for example, around fifteen (15) days, in a state that the sensor transmitter is inserted into the human body. The sensor transmitter periodically generates the blood glucose information, and a blood glucose management application is installed to the communication terminal and the communication terminal periodically receives blood glucose information from the sensor transmitter and outputs the received blood glucose information so that the user can check it.

In the continuous blood glucose monitoring system, one (1) hour to three (3) hours of a stabilization process is required when the sensor transmitter is inserted into a body portion of the user, and if the stabilization is completed, the communication terminal provides the blood glucose information received from the sensor transmitter to the user by displaying it on a display unit.

For providing accurate blood glucose information to the user, the blood glucose information received from the sensor transmitter should be calibrated at an initial stage, and should be calibrated continuously at a certain time period during a usable time period of the sensor transmitter. The blood glucose information received from the sensor transmitter should be calibrated with blood calibration information by inputting the blood calibration information measured through a separate and additional blood glucose measuring device and a strip when performing initial calibration, and after that, the blood glucose information received from the sensor transmitter should be continuously calibrated with blood calibration information measured through the blood glucose measuring device and the strip at a certain time period for a usable time period of the sensor transmitter.

The sensor transmitter and the communication terminal are continuously communicationally connected for the usable time period of the sensor transmitter and the blood glucose information measured by the sensor transmitter is provided to the communication terminal, and a case in which communication registration information of the sensor transmitter and the communication terminal is deleted or the blood glucose management application registered to the communication terminal is deleted due to the user's mistake or the sensor transmitter's and the communication terminal's operation error may occur.

When such a case occurs, a process for reconnecting communication between the sensor transmitter and the communication terminal should be performed, but the communication terminal needs to perform a stabilization process and an initial calibration process of the sensor transmitter again one more time and the repetition of those processes is unnecessary and causes the user's inconvenience.

US 2010 / 0 191 086 A1 discloses a system for sensing blood glucose data of a patient. The system includes a sensor and user interface. The sensor is calibrated using reference values stored in a reference memory. The reference values may be provided by a blood glucose measuring device. Since the reference values are kept with the sensor in the reference memory, no re-calibration is required when the sensor is reconnected to the user interface.

EP 2 327 984 A2 discloses a method for calibrating a continuous blood glucose measurement sensor.

US 2017 / 0 286 194 A1 discloses blood glucose measuring system in which a reference value for calibrating a sensor is input by a user via a user interface. US 2016 / 0 210 099 A1 discloses a further blood glucose measuring system in which a user is prompted that calibration of a sensor is pending.

### DETAILED DESCRIPTION OF INVENTION

### Technical Problem

To solve the problem of the conventional method of managing calibration information between the sensor transmitter and the communication terminal, the purpose of the present invention may be for providing a method for managing calibration information, when communication of the sensor transmitter and the communication terminal is connected, determining whether initial calibration of a sensor transmitter is performed based on a calibration identifier indicating whether initial calibration of the sensor transmitter is performed or not.

Another purpose of the present invention may be for providing a method for managing calibration information providing periodically acquired calibration information and a time point of acquiring calibration information to a sensor transmitter and, when reconnecting communication between the sensor transmitter and the communication terminal, communicationally connecting the sensor transmitter and a communication terminal without acquiring additional calibration information.

### Solution to Problem

In order to accomplish the purpose of the present invention, a method in accordance with claim 1 is provided.

According to an embodiment of the present invention, a method for managing calibration information which is used to calibrate biometric information between a sensor transmitter configured to be attachable to a part of a body of a user and a communication terminal configured to receive the biometric information from the sensor transmitter, may comprise: when connecting communication between the senser transmitter and the communication terminal, by activating an initial input interface screen on the communication terminal, acquiring, by the communication terminal, initial calibration information through the initial input interface screen; and if the initial calibration information is acquired, generating, by the communication terminal, an initial calibration message including a calibration identifier indicating whether the sensor transmitter is calibrated or not and transmitting, by the communication terminal, the generated initial calibration message to the sensor transmitter.

Preferably, the method for managing the calibration information according to an embodiment of the present invention further comprises determining whether the initial calibration information has been acquired in a process of receiving the biometric information, and the communication terminal does not provide the received biometric information received from the sensor transmitter to the user through the communication terminal until the communication terminal acquires the initial calibration information.

In the method for managing the calibration information according to an embodiment of the present invention, after the communication terminal acquires the initial calibration information, the communication terminal calibrates the biometric information received from the sensor transmitter using the initial calibration information, and provides the calibrated biometric information to the user.

The method for managing the calibration information according to the invention comprises: when connecting the communication between the sensor transmitter and the communication terminal, determining whether the sensor transmitter to be connected with the communication terminal is a sensor transmitter which was previously used; if the sensor transmitter to be connected with the communication terminal is the sensor transmitter which was previously used, receiving, from the sensor transmitter, a calibration information message including the calibration identifier indicating whether the sensor transmitter is calibrated or not; and providing to the user the biometric information received from the sensor transmitter based on the calibration information message.

In the method for managing the calibration information, when based on the calibration identifier of the calibration information message the communication terminal determines that the sensor transmitter to be connected with the communication terminal is the sensor transmitter which was previously used and calibration of the sensor transmitter was completed, the communication terminal provides to the user the biometric information received from the sensor transmitter after communication connection without stabilization of the sensor transmitter.

Preferably, the method for managing the calibration information according to the present invention may further comprise: determining whether the sensor transmitter to be connected with the communication terminal is the sensor transmitter which was previously used by comparing an identifier of the sensor transmitter received from the sensor transmitter to be connected with the communication terminal with an identifier of a sensor transmitter previously registered to the communication terminal, and determining whether a remaining usable time period of the sensor transmitter to be connected with the communication terminal is left or not based on remaining usable time period information mapped to the identifier of the sensor transmitter.

Preferably, the method for managing the calibration information according to the present invention may further comprise, when the remaining usable time period of the sensor transmitter to be connected with the communication terminal is not left, outputting an alarm message for preventing use of the sensor transmitter on the communication terminal.

Preferably, the method for managing the calibration information according to the present invention may further comprise: determining whether a set calibration period is reached or not; if the calibration period is reached, activating a periodic input interface screen on the communication terminal and periodically acquiring new calibration information through the periodic input interface screen; and if the new calibration information is acquired, transmitting, to the sensor transmitter, a periodic calibration message comprising the calibration identifier, a time point of acquiring the new calibration information, and the new calibration information.

In the method for managing the calibration information according to the present invention, the calibration information message comprises the calibration identifier, the time point of acquiring the new calibration information, and the new calibration information.

The method for managing the calibration information according to the present invention further comprises determining whether the new calibration information is needed based on difference between the time point of acquiring the new calibration information of the calibration information message and a current time, wherein when the new calibration information is needed, the communication terminal activates the periodic input interface screen on the communication terminal and acquires the new calibration information through the periodic input interface screen.

### Advantageous Effects of Invention

A method for managing calibration information according to the present invention may have various advantageous technical effect as follows.

Firstly, a method for managing calibration information according to an embodiment of the present invention communicationally connects a sensor transmitter and a communication terminal based on a calibration identifier indicating whether initial calibration of the sensor transmitter is performed or not when the sensor transmitter and the communication terminal communicationally connect to each other, thereby, in case of a sensor transmitter of which initial calibration is completed, not repeating an unnecessary stabilization process and initial calibration process and being capable of communicationally connecting the sensor transmitter and the communication terminal and being immediately usable.

Secondly, a method for managing calibration information according to an embodiment of the present invention provides periodically acquired calibration information and a time point of acquiring calibration information to a sensor transmitter and receives the calibration information and the time point of acquiring calibration information from the sensor transmitter when the communication terminal reconnects the communication, thereby determining whether additional calibration information is needed at the time of communication reconnection and acquiring additional calibration information or calibrating and outputting biometric information using the received calibration information.

### BRIEF DESCRIPTION OF THE DRAWINGS

FIG. 1 is a schematic diagram for illustrating a continuous blood glucose measurement system according to an example of the present disclosure.
FIG. 2 is a figure illustrating an applicator for attaching a sensor transmitter to a human body according to an example of the present disclosure.
FIGS. 3 and 4 are figures for illustrating a process of attaching a sensor transmitter to a human body using an applicator according to an example of the present disclosure.
FIG. 5 is a figure for illustrating messages transmitted and received between a sensor transmitter and a communication terminal.
FIG. 6 is a block diagram for illustrating a sensor transmitter according to an example of the present disclosure.
FIG. 7 is a block diagram for illustrating a communication terminal according to an example of the present disclosure.
FIG. 8 is a flowchart for illustrating a method for managing calibration information in a communication terminal according to an embodiment of the present invention.
FIG. 9 is a flowchart for illustrating a step of generating a periodic calibration message in a method for managing calibration information according to an embodiment of the present invention.
FIG. 10 is a figure for illustrating an example of an input of initial calibration information or new calibration information.
FIG. 11 illustrates an example of an initial input interface screen.
FIG. 12 illustrates an example of a periodic input interface screen.
FIG. 13 is a flowchart for illustrating a method for reconnecting communication with a sensor transmitter.
FIG. 14 is a flowchart for illustrating an example of controlling to output blood glucose information according to calibration information received through a calibration information message according to an embodiment of the present invention.
FIG. 15 illustrates an interface screen which is displayed when communication between a sensor transmitter and communication terminal is reconnected according to an embodiment of the present invention.
FIG. 16 is an interface screen which is displayed when communication reconnection of a sensor transmitter and a communication terminal is requested according to an embodiment of the present invention.

### DESCRIPTION OF EMBODIMENTS OF INVENTION

The technical terms used in the present invention are only for the purpose of describing exemplary embodiments, and they are not intended to limit the present invention. Also, unless otherwise defined, all technical terms used herein should be construed as having the same meaning as commonly understood by those skilled in the art, and should not be interpreted as being excessively inclusive or excessively restrictive. In addition, when a technical term used herein is an erroneous technical term that does not accurately represent the idea of the present invention, it should be understood as replacing the term by a technical term which can be properly understood by those skilled in the art.

Further, singular expressions used in the present specification include plural expressions unless they have definitely opposite meanings. In the present application, it shall not be construed that terms, such as "including" or "comprising", various constituent elements or steps described in the specification need to be all essentially included, and it shall be construed that some constituent elements or steps among the various constituent elements or steps may be omitted, or additional constituent elements or steps may be further included.

Also, it should be noted that the accompanying drawings are merely illustrated to easily explain the invention, and therefore, they should not be construed to limit the invention by the accompanying drawings.

Hereinafter, with reference to the enclosed drawings, a method for transmitting and receiving biometric information according to an embodiment of the present invention is described in detail.

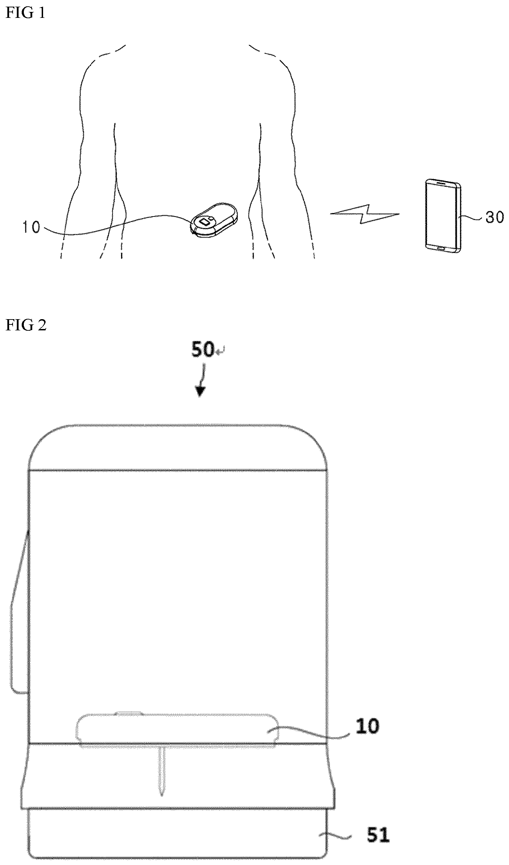

FIG. 1 is a schematic diagram for illustrating a continuous blood glucose measurement system according to an embodiment of the present invention.

Referring to FIG. 1, the continuous blood glucose measurement system (1) comprises a sensor transmitter (10) and a communication terminal (30).

The sensor transmitter (10) is attachable to human body and, when the sensor transmitter (10) is attached to the human body, an end portion of a sensor of the sensor transmitter (10) is inserted into skin to periodically extract body fluid of the human body and measure blood glucose.

The communication terminal (30) is a terminal configured to receive blood glucose information from the sensor transmitter (10) and output or display the received blood glucose information to a user, and for example, the communication terminal (30) may be a portable terminal (such as smartphone, tablet PC, or notebook and so on) configured to communicatable with the sensor transmitter (10). However, the communication terminal (30) is not limited thereto, and may be any type of a terminal which has a communication function and program or application can be installed to.

The sensor transmitter (10) transmits the blood glucose information in response to request of the communication terminal (30) or at predetermined times periodically, and for data communication between the sensor transmitter (10) and the communication terminal (30), the sensor transmitter (10) and the communication terminal (30) are communicationally connected to each other over a wire by an USB cable and so on or communicationally connected in an wireless communication means such as infrared communication, NFC communication, Bluetooth, etc.

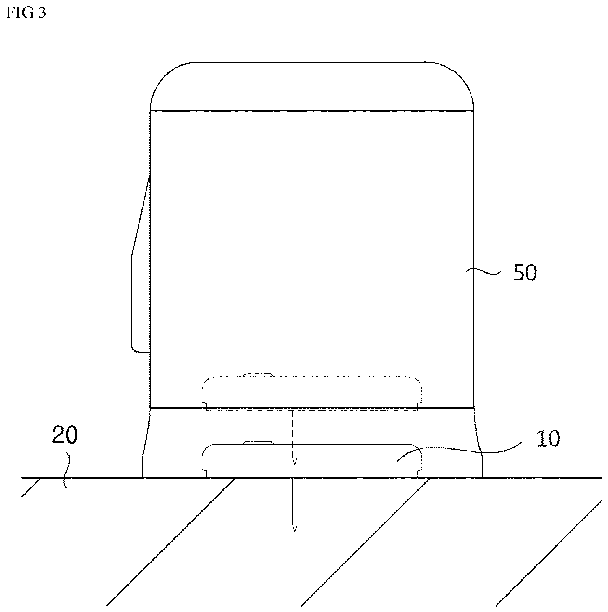

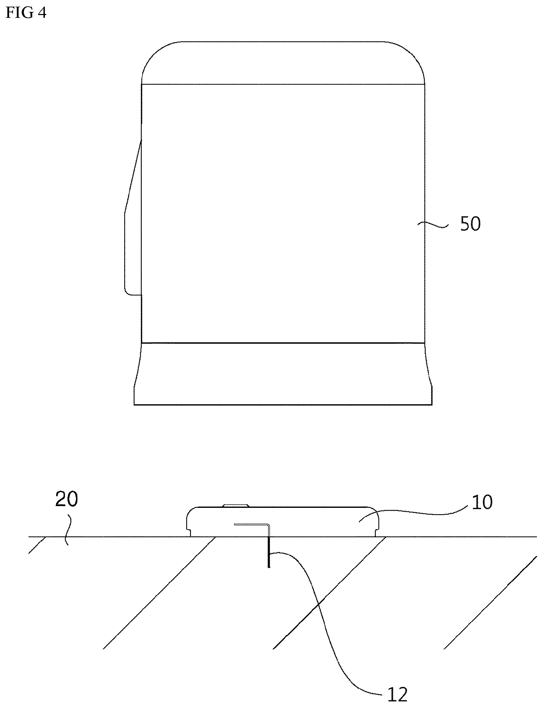

The sensor transmitter (10) is attached to a part of the human body by an applicator, FIG. 2 is a figure illustrating an applicator for attaching a sensor transmitter to a part of a human body, and FIGS. 3 and 4 are figures for illustrating a process of attaching a sensor transmitter to a human body using an applicator.

Firstly, an application (50) is now described by referring to FIG. 2, the sensor transmitter (10) is mounted in the applicator (50), and the applicator (50) can be operated so that the sensor transmitter (10) can be outwardly discharged to the outside of the applicator (50) by the manipulation of the user and then be attached to a specific portion of the human body of the user. The applicator (50) is formed to have a shape that one side of the applicator (50) is open, and the sensor transmitter (10) is installed to the applicator (50) through the open side of the applicator (50).

When the sensor transmitter (10) is attached to a part of the human body using the applicator (50), for inserting an end portion of the sensor included in the sensor transmitter (10) to skin, the applicator (50) comprises a needle (not shown) formed to cover the end portion of the sensor therein, a first elastic means (not shown) pushing the needle and the end portion of the sensor together towards the skin, and a second elastic means (not shown) configured to retract the needle only. The compressed state of the first elastic means (not shown) arranged to be compressed inside the applicator (50) by the configuration of the applicator (50) can be released, thereby inserting the needle and the end portion of the sensor simultaneously to the skin, and when the end portion of the sensor is inserted to the skin, the compressed state of the second elastic means (not shown) is released, thereby extracting the needle only. By the applicator (50), the user can safely and easily attach the sensor transmitter (10) to the skin.

A process of attaching the sensor transmitter (10) to the human body will be described in detail with reference to FIGS. 3 and 4, in a state that a protection cap (51) is separated or removed, an open side of the applicator (50) is closely placed on a specific part of skin (20) of the human body. When the applicator (50) is operated in a state that the applicator (50) is closely placed on the skin (20) of the human body, the sensor transmitter (10) is outwardly discharged from the applicator (50) and then attached to the skin (20). Here, an end portion of the sensor (12) is arranged to be exposed from the sensor transmitter (10) at a lower portion of the sensor transmitter (10), and a part of the end portion of the sensor (12) is inserted into the skin (20) by a needle installed at the applicator (50). Accordingly, the sensor transmitter (10) can be attached to the skin (20) in a state that an end portion of the sensor (12) is inserted to the skin (20).

In the example, an adhesive tape is provided at a surface of the sensor transmitter (10) contacting the human body so that the sensor transmitter (10) can be attached to the skin (20). Accordingly, if the applicator (50) is moved away from the skin (20) of the human body, the sensor transmitter (10) is fixedly attached to the skin (20) of the human body by the adhesive tape.

After that, if the power is supplied to the sensor transmitter (10), the sensor transmitter (10) is communicationally connected with the communication terminal (50), and the sensor transmitter (10) transmits the measured blood glucose information to the communication terminal (30).

The sensor transmitter (10) is stabilized for one (1) hour to three (3) hours after the communication terminal (50) is communicationally connected with the sensor transmitter (10), and the communication terminal (50) calibrates biometric information received from the sensor transmitter (10) using the calibration information inputted to the communication terminal (30) after the stabilization of the sensor transmitter (10) is completed and outputs the calibrated biometric information on the communication terminal (50) to provide it the user.

The sensor transmitter (10) can measure not only the blood glucose information but also various biometric information, and hereinafter blood glucose information is illustrated as one of examples of biometric information.

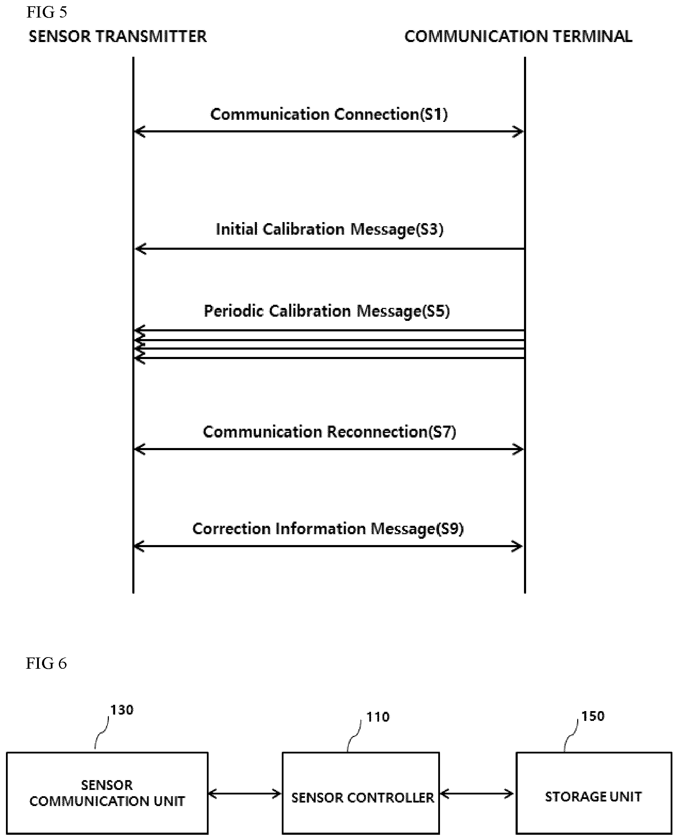

FIG. 5 is a figure for illustrating messages transmitted and received between a sensor transmitter and a communication terminal.

Referring to FIG. 5, a communication for transmitting and receiving data between the sensor transmitter and the communication terminal is connected (S1). The sensor transmitter and the communication terminal are connected via wire such as the USB cable and so on or via a wireless communication means, for example, infrared communication, NFC communication, Bluetooth, etc, but their detailed descriptions are omitted herein.

When the communication between the sensor transmitter and the communication terminal is connected, initial calibration information for calibrating blood glucose information measured by the sensor transmitter after the stabilization of the sensor transmitter is inputted to the communication terminal, and if the initial calibration information is inputted, the communication terminal generates an initial calibration message including a calibration identifier indicating whether initial calibration of the sensor transmitter is performed or not and transmits the generated initial calibration message to the sensor transmitter (S3).

New calibration information is inputted periodically for an usable time period of the sensor transmitter for accurately calibrating the blood glucose information measured by the sensor transmitter, and every time when the new calibration information is inputted, the communication terminal generates a periodic calibration message including calibration identifier, new calibration information, and time of acquiring the new calibration information, and transmits the generated periodic calibration message to the sensor transmitter (S5).

Here, when the sensor transmitter receives the initial calibration message or the periodic calibration message, the sensor transmitter stores the calibration identifier, renews calibration information by replacing previous initial calibration information or previous new calibration information with newly received new calibration information, and stores the newly received new calibration information.

Here, initial calibration information or new calibration information can be measured by a blood glucose monitoring device and a strip.

The sensor transmitter transmits the measured biometric information to the communication terminal in real time or periodically, the communication terminal calibrates the received biometric information with recent calibration information and outputs the calibrated biometric information to the user through the communication terminal, and if the communication between the sensor transmitter and the communication terminal is disconnected, or communication registration information of the communication terminal is deleted, or an blood glucose management application registered to the communication terminal is deleted, the communication between the sensor transmitter and the communication terminal is reconnected (S7).

When the communication between the sensor transmitter and the communication terminal is reconnected, the sensor transmitter transmits a calibration information message to the communication terminal (S9), and the communication terminal calibrates blood glucose information received from the sensor transmitter using a calibration identifier, initial calibration information received last time or time of acquiring new calibration information and initial calibration information or new calibration information included in the calibration information message without a stabilization and provides the calibrated blood glucose information to the user.

FIG. 6 is a block diagram for illustrating a sensor transmitter.

Referring to FIG. 6, a sensor controller (110) connects the communication with the communication terminal through the sensor communication unit (130).

When the sensor controller (110) receives an initial calibration message from the communication terminal after the communication connection, every time when a calibration identifier and initial calibration information included in the initial calibration message are stored to a storage unit (150) or a new calibration message is received, the sensor controller (110) updates the initial calibration information or the previously stored new calibration information with new calibration information included in a new calibration message and controls to store the new calibration information included in a new calibration message. Here, when receiving the initial calibration message, the sensor controller (110) controls to store information of time of acquiring initial calibration information included in the initial calibration message to the storage unit (150) or controls to renew time of acquiring new calibration information by replacing time of acquiring new calibration information previously stored with time of acquiring new calibration information included in the new calibration message.

Meanwhile, when the sensor controller (110) reconnects the communication with the communication terminal through the sensor communication unit (130) or there is a request of the communication terminal, the sensor controller (110) generates a calibration information message by extracting a calibration identifier, initial calibration information and new calibration information stored recently and information of time of acquiring initial calibration information or new calibration information stored recently, and controls to transmit the generated calibration information message to the communication terminal through the sensor communication unit (130).

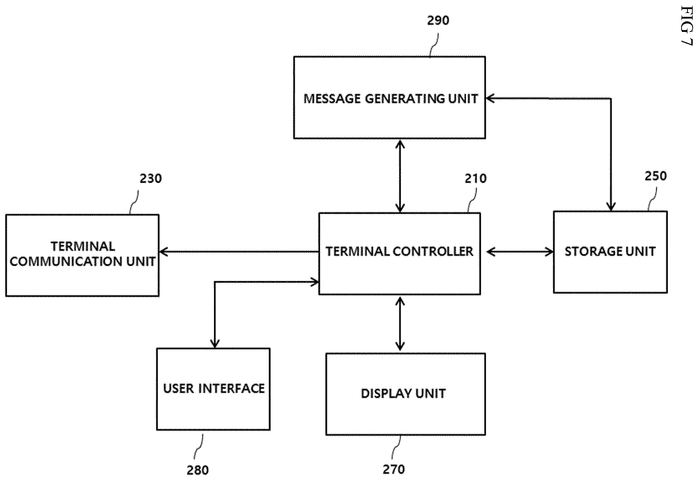

FIG. 7 is a block diagram for illustrating a communication terminal.

Referring to FIG. 7, a terminal controller (210) controls to connect the communication with the sensor transmitter through a terminal communication unit (230), and when connecting the communication, the terminal controller (210) receives an identifier of the sensor transmitter through the terminal communication unit (230) and registers and stores the received identifier of the sensor transmitter to a storage unit (250). Here, the identifier of the sensor transmitter can be used to determine whether a previously used sensor is reconnected or whether a remaining usable time period of the sensor transmitter is left.

The terminal controller (210) stabilizes the sensor transmitter for a certain time period from a time point of connecting the communication with the sensor transmitter, and when the stabilization of the sensor transmitter is completed, displays an initial input interface display through a display unit (270). When initial calibration information is inputted through a user interface unit (280), the terminal controller (210) controls to store the inputted initial calibration information and information of acquiring the initial calibration information to the storage unit (250).

The terminal controller (210) counts use time of the sensor transmitter from a time point of connecting the communication with the sensor transmitter, when a set calibration period reaches, periodically controls to display a period input interface screen for acquiring new calibration information on a display unit (270), and, when new calibration information is inputted through the user interface (280), controls to store information of time of acquiring new calibration information to the storage unit (250).

Meanwhile, when initial calibration information is inputted, a message generating unit (290) generates an initial calibration message including a calibration identifier indicating that initial calibration of the sensor transmitter was performed, initial calibration information and time of acquiring initial calibration information, or when new calibration information is inputted, generates a periodic calibration message including a calibration identifier, new calibration information, and time of acquiring new calibration information. The terminal controller (210) controls to transmit the generated initial calibration message or periodic calibration information to the sensor transmitter through the terminal communication unit (230).

The terminal controller (210) compares an identifier of the sensor transmitter, received from the sensor transmitter when connecting the communication with the sensor transmitter, with an identifier of the sensor transmitter, previously stored to the storage unit (250), determines whether a sensor transmitter to connect communication is a previously used sensor transmitter, and if a sensor transmitter to connect communication is a previously used sensor transmitter, whether a remaining useable time period is left, and controls the communication connection with the sensor transmitter, or when it is determined that a sensor transmitter to connect communication is a previously used sensor transmitter based on the calibration information message received from the sensor transmitter, controls to provide the calibrated blood glucose information to the user based on whether initial calibration is performed or whether acquired initial calibration information or new calibration information exists within a threshold range set based on current time.

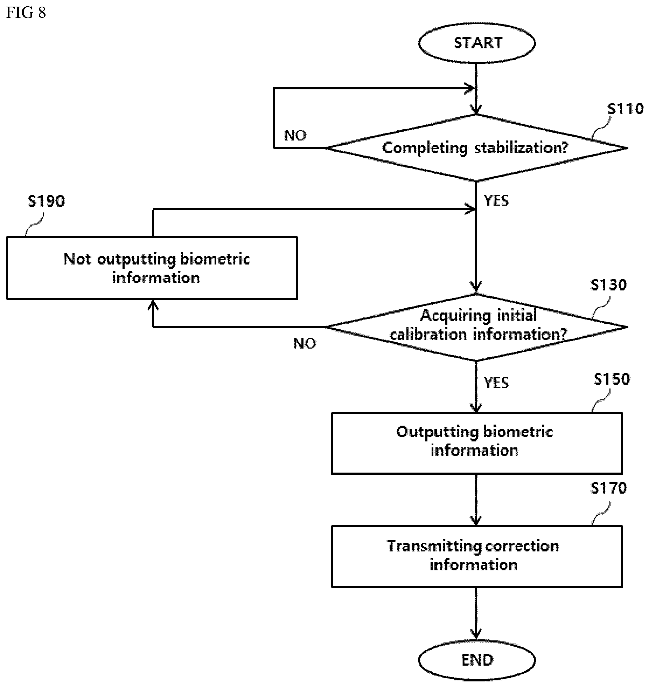

FIG. 8 is a flowchart for illustrating a method for managing calibration information in a communication terminal according to an embodiment of the present invention.

Referring to FIG. 8, the communication terminal determines whether stabilization of the sensor transmitter is completed after the communication connection with the sensor transmitter (S110).

When the stabilization of the sensor transmitter is completed, whether initial calibration information is acquired through an initial input interface screen is determined (S130). Until the initial calibration information is acquired, even though blood glucose information is received from the sensor transmitter, the received blood glucose information is not provided to the user (S190), but after the initial calibration information is acquired, the blood glucose information received from the sensor transmitter is calibrated with initial calibration information and the calibrated biometric information is outputted through a display unit to provide it to the user (S150).

Meanwhile, when the initial calibration information is acquired, an initial calibration message including a calibration identifier indicating that initial calibration of the sensor transmitter was performed, the acquired initial calibration information, and time of acquiring the initial calibration information is generated or a periodic calibration message including new calibration information acquired periodically, time of acquiring the new calibration information, and a calibration identifier is generated, and the calibrated initial calibration message or periodic calibration message is transmitted to the sensor transmitter (S170).

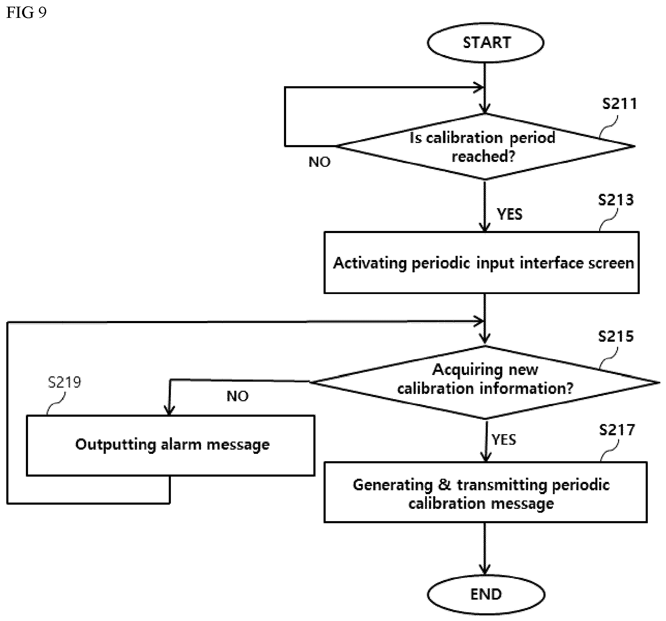

FIG. 9 is a flowchart for illustrating a step of generating a periodic calibration message in a method for managing calibration information according to an embodiment of the present invention.

Referring to FIG. 9, after communication between the sensor transmitter and the communication terminal is connected, the user time of the sensor transmitter is counted to determine whether set calibration period is reached (S211).

When the set calibration period is reached, a periodic input interface screen is activated on a display unit for acquiring new calibration information. According to a field to which the present invention is applied, an alarm sound or vibration for requesting to input new calibration information can be outputted instead of a periodic input interface screen in order to acquire new calibration information.

By determining whether new calibration information is inputted through a user interface, when the new calibration information is inputted, a periodic calibration message including new calibration information, a time point of acquiring the new calibration information and a calibration identifier is generated, and the generated periodic calibration message is transmitted to the sensor transmitter (S217).

However, when new calibration information is not inputted even though set input time passes, an alarm message is outputted to the user (S219). Here, the alarm message is a message inducing the user to input new calibration information, and the alarm message can be displayed on a display or be outputted as an alarm sound or vibration.

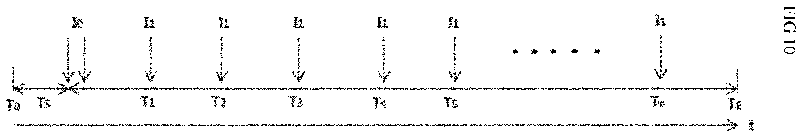

FIG. 10 is a figure for illustrating an example of an input of initial calibration information or new calibration information, and referring to FIG. 10, the sensor transmitter is stabilized until a set stabilization time period (T _{S}) from a time point (T ₀) of connecting the communication between the sensor transmitter and the communication terminal passes.

When the stabilization of the sensor transmitter is completed, initial calibration information (I ₀) is inputted. Here, the initial calibration information (I ₀) can be inputted multiple times for accurately calibrating biometric information of the sensor transmitter.

After the stabilization of the sensor transmitter is completed, new calibration information (I ₁, I ₂, I ₃, I ₄....) is periodically inputted, preferably every twelve (12) hours or every day, until a time point of expiration of a usable time period of the sensor transmitter, and blood glucose information received from the sensor transmitter is calibrated with the new calibration information and the calibrated blood glucose information is provided to the user.

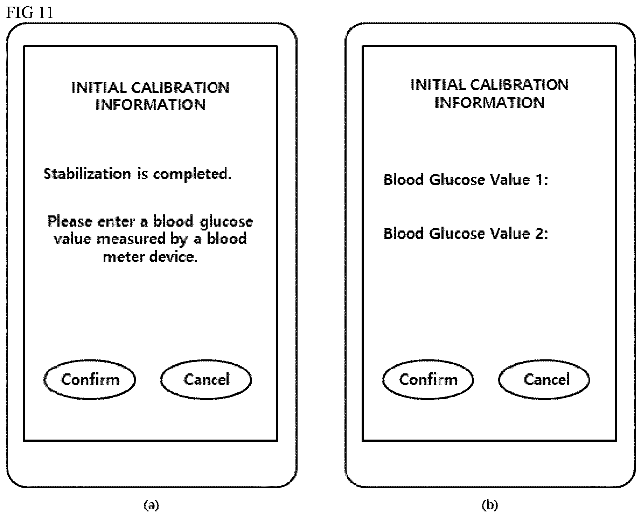

FIG. 11 illustrates an example of an initial input interface screen.

As illustrated in FIG. 11(a), an initial input interface screen for notifying that the stabilization of the sensor transmitter was completed and requesting to input initial calibration information is displayed. When the user selects an icon (Confirm) for inputting initial calibration information, an initial input interface screen for inputting initial calibration information is displayed as illustrated in FIG. 11(b).

The user inputs a blood glucose value measured by a blood glucose meter device (i.e. initial calibration information) through an initial input interface screen for inputting initial calibration information.

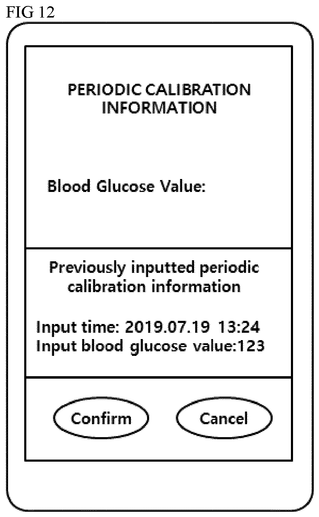

FIG. 12 illustrates an example of a periodic input interface screen.

As illustrated in FIG. 12, when a calibration period is reached, a period input interface screen for inputting new calibration information is displayed. The user inputs a blood glucose value measured by a blood glucose measurement device (i.e. new calibration information) through a periodic input interface screen for inputting new calibration information.

Preferably, previously acquired new calibration information and information of a time point of acquiring new calibration information are displayed together on the periodic calibration interface screen.

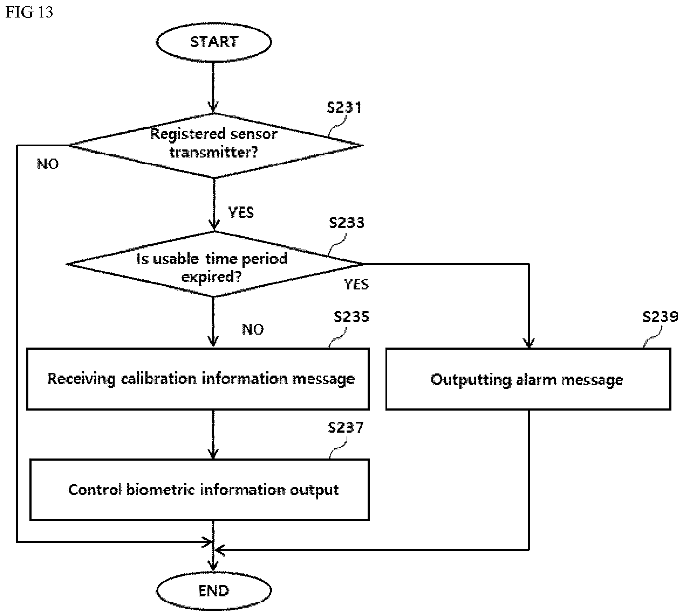

FIG. 13 is a flowchart for illustrating a method for reconnecting communication with a sensor transmitter.

Referring to FIG. 13, when the communication terminal is communicationally connected with the sensor transmitter, the communication terminal receives an identifier of the sensor transmitter from the sensor transmitter, and determines whether a sensor transmitter to connect communication is a sensor transmitter which is registered due to previous use by comparing the received identifier of the sensor transmitter with an identifier of the sensor transmitter registered to the communication terminal (S231).

When the sensor transmitter to connect communication is a registered sensor transmitter, whether the usable time period of the sensor transmitter is expired is determined based on information of a remaining usable time period of the sensor transmitter mapped to the identifier of the sensor transmitter (S233). Here, the remaining usable time period of the sensor transmitter can be calculated by subtracting a time period from a time point of initial communication connection between the sensor transmitter and the communication terminal to a current time point from a whole usable time period of the sensor transmitter.

When the usable time period of the sensor transmitter is expired, the sensor transmitter cannot be used anymore, and an warning message for recommending using a new sensor transmitter is outputted on a display unit (S239).

However, when the usable time period of the sensor transmitter is left, a calibration information message is received from the sensor transmitter (S235). The calibration information message comprises a calibration identifier, recently received initial calibration information or new calibration information, and a time point of acquiring recently received initial calibration information or new calibration information.

The blood glucose information received from the sensor transmitter is calibrated with the initial calibration information or new calibration information of the calibration information message, and the calibrated blood glucose information is outputted on a display unit to provide it to the user (S237).

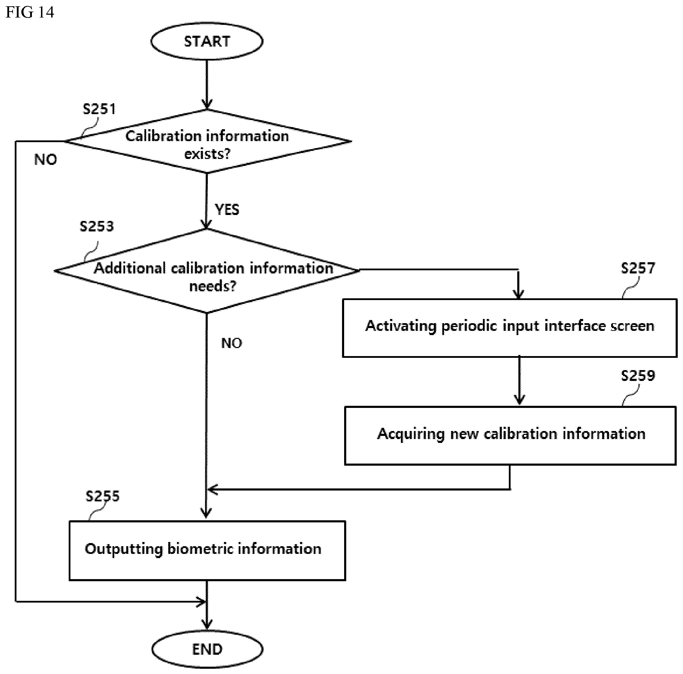

FIG. 14 is a flowchart for illustrating an example of controlling to output blood glucose information according to calibration information received through a calibration information message according to an embodiment of the present invention.

Referring to FIG. 14, whether a calibration information message received from the sensor transmitter when connecting the communication with the sensor transmitter includes initial calibration information or new calibration information is determined (S251).

When the initial calibration information or new calibration information exists, whether additional calibration information is needed is determined based on a time point of acquiring the initial calibration information or new calibration information (S253). When the time point of acquiring the initial calibration information or new calibration information is within a threshold time period according to a current time point (i.e. the time point of acquiring the initial calibration information or new calibration information is within a next calibration period set by recent acquirement according to the current time point), it is determined that the additional calibration information is not needed.

When the additional calibration information is not needed, the blood glucose information received from the sensor transmitter is calibrated using the received initial calibration information or new calibration information, and the calibrated blood glucose information is provided to the user (S255).

However, if the additional calibration information is needed, a periodic input interface screen for acquiring the additional calibration information is activated (S257), and the new calibration information is acquired through the activated periodic input interface screen (S259).

When the new calibration information is acquired, a next calibration period is reckoned from a time point of acquiring new calibration information with additional calibration information.

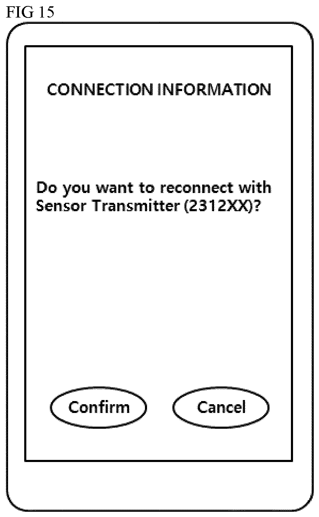

FIG. 15 illustrates an interface screen which is displayed when communication between a sensor transmitter and communication terminal is reconnected according to an embodiment of the present invention, and an identifier (2312XX) of the sensor transmitter received from the sensor transmitter is displayed when the communication with the sensor transmitter is reconnected.

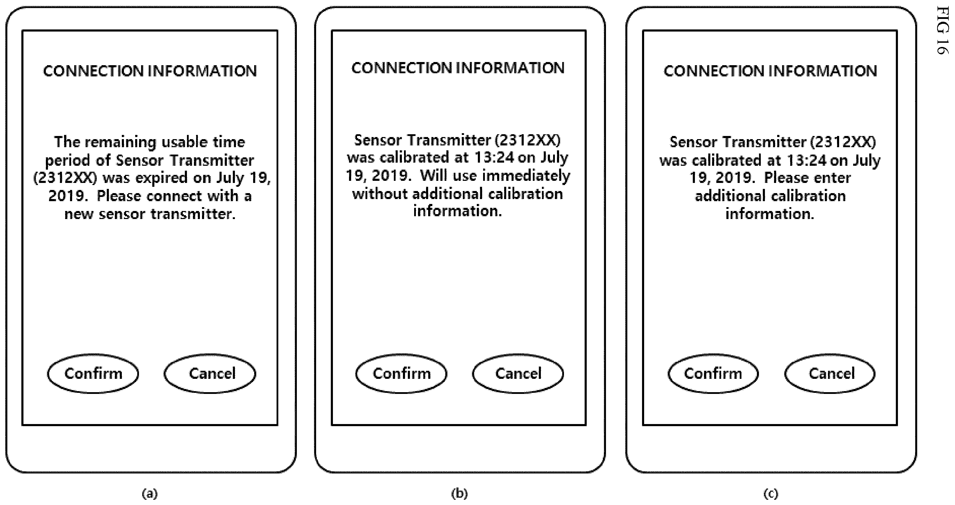

FIG. 16 is an interface screen which is displayed when communication reconnection of a sensor transmitter and a communication terminal is requested according to an embodiment of the present invention.

As illustrated in FIG. 16(a), when it is determined that a remaining usable period of the sensor transmitter is expired based on an identifier of the sensor transmitter which is requested to reconnect the communication with the communication terminal, an alarm message suggesting to replace a new sensor transmitter is outputted.

Referring to FIG. 16(b), when based on a calibration information message received from the communicationally reconnected sensor transmitter it is determined that no additional calibration information is not needed, a message indicating that the sensor transmitter can be used immediately without additional calibration information is outputted. However, when the user requests to input additional calibration information (e.g. when selecting a "Cancel" icon) even though the user can use without the additional calibration information, a periodic input interface screen is activated on a display unit. Even in the case that additional calibration information is not needed, blood glucose information calibrated by inputting new calibration information again can be provided for accuracy.

Referring to FIG. 16(c), when based on the calibration information message received from the communicationally reconnected sensor transmitter it is determined that additional calibration information is needed, a message indicating that additional calibration information is needed. When the user requests to input additional calibration information (e.g. when selecting "Confirm" icon), a periodic input interface screen is activated on a display unit.

The embodiments of the present invention may be written as computer programs and may be implemented in general-use digital computers that execute the programs using a computer readable recording medium.

Examples of the computer readable recording medium include magnetic storage media (e.g., ROM, floppy disks, hard disks, etc.), optical recording media (e.g., CD-ROMs, or DVDs) and carrier wave (e.g. transmission through the Internet).

Although the present invention is described with exemplary embodiments illustrated in the drawings, the foregoing descriptions have been presented by way of example, and a person having ordinary skill in the art which the present invention relates could make various modifications. Accordingly, it should be understood that the scope of the present invention shall be defined by the Claims.

## Claims

1. A method for managing calibration information which is used to calibrate biometric information between a sensor transmitter (10) configured to be attachable to a part of a body of a user and a communication terminal (30) configured to receive the biometric information from the sensor transmitter (10), the method comprising:
upon connecting communication between the sensor transmitter (10) and the communication terminal (30), determining whether the sensor transmitter (10) to be connected with the communication terminal (30) is a sensor transmitter (10) which was previously used;
if the sensor transmitter (10) was previously used, receiving, from the sensor transmitter (10), a calibration information message including a calibration identifier indicating whether the sensor transmitter (10) is calibrated or not, calibration information and a time of acquiring the calibration information;
determining whether additional calibration information is needed or not based on the time of acquiring the calibration information and a current time point;
when it is determined that additional calibration information is needed, activating a periodic input interface screen for acquiring the additional calibration information on the communication terminal (30);
wherein the biometric information is calibrated using the calibration information when the time of acquiring the calibration information is within a threshold time period according to the current time point, and
wherein the biometric information is calibrated using the additional calibration information when the time of acquiring the calibration information is out of the threshold time period according to the current time point.

2. The method for managing the calibration information according to claim 1, further comprising:
determining whether an initial calibration information has been acquired in a process of receiving the biometric information, wherein the communication terminal (30) provides to the user the biometric information received from the sensor transmitter (10) after the initial calibration information is acquired.

3. The method for managing the calibration information according to claim 2, wherein, after the communication terminal (30) acquires the initial calibration information, the communication terminal (30) calibrates the biometric information received from the sensor transmitter (10) using the initial calibration information, and provides the calibrated biometric information to the user.

4. The method for managing the calibration information according to any one claims 1 to 3, wherein, when based on the calibration identifier of the calibration information message the communication terminal (30) determines that the sensor transmitter (10) to be connected with the communication terminal (30) is the sensor transmitter (10) which was previously used and calibration of the sensor transmitter (10) was completed, the communication terminal (30) provides to the user the biometric information received from the sensor transmitter (10) after communication connection without stabilization of the sensor transmitter (10).

5. The method for managing the calibration information according to any one of claims 1 to 3, further comprising:
determining whether the sensor transmitter (10) to be connected with the communication terminal (30) is the sensor transmitter (10) which was previously used includes comparing an identifier of the sensor transmitter (10) received from the sensor transmitter (10) to be connected with the communication terminal (30) with an identifier of a sensor transmitter (10) previously registered to the communication terminal (30), and
determining whether a remaining usable time period of the sensor transmitter (10) to be connected with the communication terminal (30) is left or not based on remaining usable time period information mapped to the identifier of the sensor transmitter (10).

6. The method for managing the calibration information according to claim 5, further comprising, when the remaining usable time period of the sensor transmitter (10) to be connected with the communication terminal (30) is not left, outputting an alarm message for preventing use of the sensor transmitter (10) on the communication terminal (30).

## Patentansprüche

1. Verfahren zum Verwalten von Kalibrierungsinformationen, die zum Kalibrieren biometrischer Informationen zwischen einem Sensorsender (10), welcher derart konfiguriert ist, dass er an einem Teil eines Körpers eines Benutzers anbringbar ist, und einem Kommunikationsendgerät (30), das zum Empfangen der biometrischen Informationen von dem Sensorsender (10) konfiguriert ist, verwendet werden, wobei das Verfahren umfasst:
beim Herstellen einer Kommunikationsverbindung zwischen dem Sensorsender (10) und dem Kommunikationsendgerät (30), Bestimmen, ob der mit dem Kommunikationsendgerät (30) zu verbindende Sensorsender (10) ein Sensorsender (10) ist, der zuvor verwendet wurde;
falls der Sensorsender (10) zuvor verwendet wurde, Empfangen einer Kalibrierungsinformationsnachricht von dem Sensorsender (10), die eine Kalibrierungskennung, die angibt, ob der Sensorsender (10) kalibriert ist oder nicht, Kalibrierungsinformationen und einen Zeitpunkt der Erfassung der Kalibrierungsinformationen enthält;
Bestimmen, ob zusätzliche Kalibrierungsinformationen benötigt werden oder nicht, auf der Grundlage des Zeitpunkts der Erfassung der Kalibrierungsinformationen und eines aktuellen Zeitpunkts;
wenn bestimmt wird, dass zusätzliche Kalibrierungsinformationen benötigt werden, Aktivieren eines periodischen Eingabeschnittstellenbildschirms zum Erfassen der zusätzlichen Kalibrierungsinformationen auf dem Kommunikationsendgerät (30);
wobei die biometrischen Informationen unter Verwendung der Kalibrierungsinformationen kalibriert werden, wenn der Zeitpunkt der Erfassung der Kalibrierungsinformationen bezogen auf den aktuellen Zeitpunkt innerhalb eines Schwellenzeitraums liegt, und
wobei die biometrischen Informationen unter Verwendung der zusätzlichen Kalibrierungsinformationen kalibriert werden, wenn der Zeitpunkt der Erfassung der Kalibrierungsinformationen bezogen auf den aktuellen Zeitpunkt außerhalb des Schwellenzeitraums liegt.

2. Verfahren zum Verwalten der Kalibrierungsinformationen nach Anspruch 1, ferner umfassend:
Bestimmen, ob in einem Vorgang des Empfangens der biometrischen Informationen anfängliche Kalibrierungsinformationen erfasst worden sind, wobei das Kommunikationsendgerät (30) dem Benutzer die von dem Sensorsender (10) empfangenen biometrischen Informationen bereitstellt, nachdem die anfänglichen Kalibrierungsinformationen erfasst worden sind.

3. Verfahren zum Verwalten der Kalibrierungsinformationen nach Anspruch 2, wobei, nachdem das Kommunikationsendgerät (30) die anfänglichen Kalibrierungsinformationen erfasst hat, das Kommunikationsendgerät (30) die von dem Sensorsender (10) empfangenen biometrischen Informationen unter Verwendung der anfänglichen Kalibrierungsinformationen kalibriert und dem Benutzer die kalibrierten biometrischen Informationen bereitstellt.

4. Verfahren zum Verwalten der Kalibrierungsinformationen nach einem der Ansprüche 1 bis 3, wobei, wenn das Kommunikationsendgerät (30) auf der Grundlage der Kalibrierungskennung der Kalibrierungsinformationsnachricht bestimmt, dass der mit dem Kommunikationsendgerät (30) zu verbindende Sensorsender (10) der Sensorsender (10) ist, der zuvor verwendet wurde, und dass die Kalibrierung des Sensorsenders (10) abgeschlossen wurde, das Kommunikationsendgerät (30) dem Benutzer die von dem Sensorsender (10) empfangenen biometrischen Informationen nach dem Herstellen der Kommunikationsverbindung ohne Stabilisierung des Sensorsenders (10) bereitstellt.

5. Verfahren zum Verwalten der Kalibrierungsinformationen nach einem der Ansprüche 1 bis 3, ferner umfassend:
Bestimmen, ob der mit dem Kommunikationsendgerät (30) zu verbindende Sensorsender (10) der Sensorsender (10) ist, der zuvor verwendet wurde, wobei das Bestimmen ein Vergleichen einer von dem mit dem Kommunikationsendgerät (30) zu verbindenden Sensorsender (10) empfangenen Kennung des Sensorsenders (10) mit einer Kennung eines zuvor bei dem Kommunikationsendgerät (30) registrierten Sensorsenders (10) umfasst, und
Bestimmen, ob eine verbleibende Nutzungsdauer des mit dem Kommunikationsendgerät (30) zu verbindenden Sensorsenders (10) vorhanden ist oder nicht, auf der Grundlage von Informationen über die verbleibende Nutzungsdauer, die der Kennung des Sensorsenders (10) zugeordnet sind.

6. Verfahren zum Verwalten der Kalibrierungsinformationen nach Anspruch 5, ferner umfassend, wenn die verbleibende Nutzungsdauer des mit dem Kommunikationsendgerät (30) zu verbindenden Sensorsenders (10) nicht mehr vorhanden ist, das Ausgeben einer Alarmmeldung zum Verhindern der Verwendung des Sensorsenders (10) auf dem Kommunikationsendgerät (30).

## Revendications

1. Procédé de gestion d'informations d'étalonnage, utilisé pour étalonner des informations biométriques entre un émetteur de détection (10) prévu pour pouvoir être fixé sur une partie du corps d'un utilisateur et un terminal de communication (30) prévu pour recevoir les informations biométriques en provenance de l'émetteur de détection (10), ledit procédé comprenant :
la détermination, à l'établissement d'une communication entre l'émetteur de détection (10) et le terminal de communication (30), si l'émetteur de détection (10) à connecter au terminal de communication (30) est un émetteur de détection (10) précédemment utilisé ;
si l'émetteur de détection (10) a été précédemment utilisé, la réception, en provenance de l'émetteur de détection (10), d'un message d'informations d'étalonnage comprenant un identifiant d'étalonnage indiquant si l'émetteur de détection (10) est étalonné ou non, des informations d'étalonnage et le moment d'acquisition des informations d'étalonnage ;
la détermination si des informations d'étalonnage additionnelles sont exigées ou non, sur la base du moment d'acquisition des informations d'étalonnage et d'un moment actuel ;
s'il est déterminé que des informations d'étalonnage additionnelles sont exigées, l'activation d'un écran d'interface d'entrée périodique pour acquérir les informations d'étalonnage additionnelles sur le terminal de communication (30) ;
où les informations biométriques sont étalonnées au moyen des informations d'étalonnage si le moment d'acquisition des informations d'étalonnage est compris dans une période seuil en fonction du moment actuel, et
où les informations biométriques sont étalonnées au moyen des informations d'étalonnage additionnelles si le moment d'acquisition des informations d'étalonnage est en dehors de la période seuil en fonction du moment actuel.

2. Procédé de gestion des informations d'étalonnage selon la revendication 1, comprenant en outre :
la détermination si une information d'étalonnage initiale a été acquise lors d'un processus de réception des informations biométriques, le terminal de communication (30) fournissant à l'utilisateur les informations biométriques reçues en provenance de l'émetteur de détection (10) après que les informations d'étalonnage initiales ont été acquises.

3. Procédé de gestion des informations d'étalonnage selon la revendication 2, où, après que le terminal de communication (30) a acquis les informations d'étalonnage initiales, le terminal de communication (30) étalonne les informations biométriques reçues de l'émetteur de détection (10) au moyen des informations d'étalonnage initiales, et fournit les informations biométriques étalonnées à l'utilisateur.

4. Procédé de gestion des informations d'étalonnage selon l'une des revendications 1 à 3, où, sur la base de l'identifiant d'étalonnage du message d'informations d'étalonnage, le terminal de communication (30) détermine que l'émetteur de détection (10) à connecter au terminal de communication (30) est l'émetteur de détection (10) précédemment utilisé, et un étalonnage de l'émetteur de détection (10) a été exécuté, le terminal de communication (30) fournit à l'utilisateur les informations biométriques reçues en provenance de l'émetteur de détection (10) après établissement de communication sans stabilisation de l'émetteur de détection (10).

5. Procédé de gestion des informations d'étalonnage selon l'une des revendications 1 à 3, comprenant en outre :
la détermination si l'émetteur de détection (10) à connecter au terminal de communication (30) est l'émetteur de détection (10) précédemment utilisé comprend la comparaison d'un identifiant de l'émetteur de détection (10) reçu en provenance de l'émetteur de détection (10) à connecter au terminal de communication (30) avec un identifiant d'un émetteur de détection (10) précédemment enregistré auprès du terminal de communication (30), et
la détermination si une période utilisable de l'émetteur de détection (10) à connecter au terminal de communication (30) est restante ou non sur la base d'informations de période utilisable restante associées à l'identifiant de l'émetteur de détection (10).

6. Procédé de gestion des informations d'étalonnage selon la revendication 5, comprenant en outre, si une période utilisable de l'émetteur de détection (10) à connecter au terminal de communication (30) n'est pas restante, l'émission d'un message d'alarme pour empêcher l'utilisation de l'émetteur de détection (10) sur le terminal de communication (30).
